## Europäisches Patentamt
## European Patent Office
## Office européen des brevets

(19)

(11) Veröffentlichungsnummer: **0 198 321**
**A1**

(12)

# EUROPÄISCHE PATENTANMELDUNG

(21) Anmeldenummer: 86104475.8

(22) Anmeldetag: 02.04.86

(51) Int. Cl.⁴: **A 61 K 37/54**
// (A61K37/54, 31:725)

(30) Priorität: 11.04.85 DE 3512908

(43) Veröffentlichungstag der Anmeldung: 22.10.86
Patentblatt 86/43

(84) Benannte Vertragsstaaten: **AT BE CH DE FR GB IT LI LU NL SE**

(71) Anmelder: **BEHRINGWERKE AKTIENGESELLSCHAFT, Postfach 1140, D-3550 Marburg/Lahn (DE)**

(72) Erfinder: **Pâques, Eric Paul, Schmiedeacker 18, D-3550 Marburg (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) Einen Plasminogenaktivator enthaltendes Mittel.

(57) Es wird ein Arzneimittel, das einen Plasminogenaktivator aus der Gruppe bestehend aus Gewebe-Plasminogenaktivator und seinen natürlich vorkommenden oder synthetisch oder gentechnisch hergestellten Derivaten und einen Polyschwefelsäureester eines Saccharids oder einen sulfatierten Zucker enthält, ein Verfahren zur Herstellung eines solchen Arzneimittels sowie seine Verwendung zur Behandlung von Thrombosen und Embolien beschrieben.

EP 0 198 321 A1

I

## Einen Plasminogenaktivator enthaltendes Mittel

Die Erfindung betrifft ein den Gewebe-Plasminogenaktivator oder eines seiner natürlich vorkommenden,
synthetisch oder gentechnisch hergestellten Derivate
enthaltendes Arzneimittel.

Die Gruppe umfassend den Gewebe-Plasminogenaktivator
(t-PA) und seine natürlich vorkommenden, synthetischen
oder gentechnisch hergestellten Derivate wird hier als
Plasminogenaktivatoren (PA) bezeichnet. Plasminogen wird
durch Plasminogenaktivatoren in Plasmin umgewandelt. Zu
den Katalysatoren dieser Reaktion zählen Streptokinase,
Urokinase und t-PA. Die therapeutische Anwendung dieser
Aktivatoren als Fibrinolytika ist bekannt. Aufgrund
seiner hohen Affinität zu Fibrin ist t-PA für die Lyse-
Therapie von besonderer Bedeutung.

Der vorliegenden Erfindung liegt die Aufgabe zugrunde,
eine PA-Präparation herzustellen, welche wirksamer als
reiner Gewebe-Plasminogenaktivator ist.

Es wurde überraschenderweise gefunden, daß die oben
definierten Plasminogenaktivatoren (PA) eine hohe Affinität zu Polyschwefelsäureestern von Sacchariden oder
sulfatierten Zuckern aufweist, und daß die Aktivität von
PA in Anwesenheit eines Polyschwefelsäureester eines
Saccharids oder sulfatierten Zuckers gesteigert ist.

Gegenstand der Erfindung ist deshalb ein Arzneimittel,
enthaltend eine Mischung aus einem Plasminogenaktivator
aus der Gruppe bestehend aus Gewebe-Plasminogenaktivator
und seinen natürlich vorkommenden, synthetisch oder gentechnisch hergestellten Derivaten (PA) und einem Polyschwefelsäureester eines Saccharids oder sulfatierten Zucker.

Ein solches Arzneimittel wird vorzugsweise zur Behandlung von Thrombosen und Embolien verwendet.

Bevorzugt enthält ein solches Arzneimittel einen PA und einen Polyschwefelsäureester eines Saccharids oder sulfatierten Zucker in einem molaren Verhältnis von 10:1 bis 1:100.000, vorzugsweise 10:1 bis 1:10.000.

Die Bindung von t-PA an Polyschwefelsäureester von Sacchariden oder sulfatierte Zucker konnte mittels Immunelektrophorese gezeigt werden. T-PA wurde mit Heparin, Heparan-Sulfat, Anteparon , Dextran-Sulfat oder Pentosan-Sulfat inkubiert und immunelektrophoretisch untersucht. Die Bande des mit einem Polyschwefelsäureester eines Saccharids oder sulfatierten Zucker inkubierten t-PA verschob sich in anodischer Richtung, während t-PA fast an der Auftragsstelle blieb.

Gegenstand der Erfindung ist auch ein Verfahren zur Herstellung eines solchen Arzneimittels, dadurch gekennzeichnet, daß t-PA oder eines seiner natürlich vorkommenden, synthetisch oder gentechnisch hergestellten Derivate mit einem Polyschwefelsäureester eines Saccharids oder sulfatierten Zucker, vorzugsweise in einem molaren Verhältnis von 10:1 bis 1:100.000, besonders bevorzugt in einem Verhältnis von 10:1 bis 1:10.000, und gegebenenfalls einem Stabilisator versetzt, gegebenenfalls der Überschuß an verwendetem Polyschwefelsäureester oder sulfatierten Zucker abgetrennt und gegebenenfalls gefriergetrocknet wird.

Als Stabilisator können beispielsweise Albumin, Gelatine oder ein abgebautes, chemisch vernetztes Kollagen oder eine solche Gelatine verwendet werden.

<u>Patentansprüche</u>:

1. Arzneimittel enthaltend eine Mischung aus einem Plasminogenaktivator (PA) aus der Gruppe bestehend aus Gewebe-Plasminogenaktivator und seinen natürlich vorkommenden oder synthetisch oder gentechnisch hergestellten Derivaten und einem Polyschwefelsäureester eines Saccharids oder sulfatierten Zucker und gegebenenfalls einen Stabilisator.

2. Arzneimittel nach Anspruch 1 enthaltend Human-t-PA.

3. Arzneimittel nach Anspruch 1 enthaltend Heparin.

4. Arzneimittel nach Anspruch 1 enthaltend t-PA und einen Polyschwefelsäureester eines Saccharid oder sulfatierten Zucker in einem molaren Verhältnis von 10:1 bis 1:100.000, vorzugsweise 10:1 bis 1:10.000.

5. Arzneimittel nach Anspruch 1 enthaltend t-PA und Heparin in einem molaren Verhältnis von 10:1 bis 1:100.000, vorzugsweise 10:1 bis 1:10.000.

6. Arzneimittel nach Anspruch 1 enthaltend Albumin, Gelatine oder ein abgebautes, chemisch vernetztes Kollagen oder eine abgebaute, chemisch vernetzte Gelatine.

7. Verfahren zur Herstellung eines Arzneimittels nach Anspruch 1, dadurch gekennzeichnet, daß ein Plasminogenaktivator (PA) aus der Gruppe bestehend aus Gewebe-plasminogenaktivator und seinen natürlich vorkommenden, synthetisch oder gentechnisch hergestellten Derivaten mit einem Polyschwefelsäureester eines Saccharids oder sulfatierten Zucker, vorzugsweise in einem molaren Verhältnis von 10:1 bis 1:100.000, besonders bevorzugt in einem Verhältnis von 10:1 bis 1:10.000, und gegebenenfalls einem Stabilisator versetzt und gegebenenfalls gefriergetrocknet wird.

8. Verwendung eines Arzneimittels nach Anspruch 1 für die Behandlung von Thrombosen oder Embolien.

**Europäisches Patentamt**

**EUROPÄISCHER RECHERCHENBERICHT**

0198321
Nummer der Anmeldung

EP 86 10 4475

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CH-A- 630 661 (PIERRE FABRE S.A.)<br>* Seite 2, linke Spalte, Zeilen 1-45 (Ansprüche 1-7) *<br>--- | 1-8 | A 61 K 37/54 //<br>(A 61 K 37/54<br>A 61 K 31:725) |
| X | FR-A-2 310 133 (PIERRE FABRE S.A.)<br>* Seite 12, Zeile 1 - Seite 13, Zeile 19 (Ansprüche 1-10) *<br>--- | 1-8 | |
| X | EP-A-0 041 449 (PIERRE FABRE S.A.)<br>* Seite 6, Zeilen 14-16 (Anspruch 4) *<br>----- | 1-8 | |
| | | | **RECHERCHIERTE SACHGEBIETE (Int. Cl.4)**<br><br>A 61 K |

Der vorliegende Recherchenbericht wurde für alle Patentansprüche erstellt.

| Recherchenort | Abschlußdatum der Recherche | Prüfer |
|---|---|---|
| DEN HAAG | 22-07-1986 | BRINKMANN C. |